(19) Europäisches Patentamt / European Patent Office / Office européen des brevets

(11) **EP 3 432 847 B1**

(12) **EUROPEAN PATENT SPECIFICATION**

(45) Date of publication and mention of the grant of the patent:
**22.01.2020 Bulletin 2020/04**

(21) Application number: **17713295.8**

(22) Date of filing: **24.03.2017**

(51) Int Cl.:
*A61F 9/008* (2006.01)        *A61B 18/20* (2006.01)
*G02F 1/37* (2006.01)        *A61B 18/00* (2006.01)

(86) International application number:
**PCT/EP2017/057065**

(87) International publication number:
**WO 2017/167657 (05.10.2017 Gazette 2017/40)**

(54) **MEDICAL LASER SYSTEM**

MEDIZINISCHES LASERSYSTEM

SYSTÈME LASER MÉDICAL

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **26.03.2016 DK 201600181**
**24.11.2016 DK 201670935**

(43) Date of publication of application:
**30.01.2019 Bulletin 2019/05**

(73) Proprietor: **Norlase Aps**
**2750 Ballerup (DK)**

(72) Inventors:
• **FLANDERS, Dale**
 **Lexington**
 **Massachusetts 02420-3226 (US)**
• **SKOVGAARD, Peter**
 **3460 Birkerød (DK)**
• **HANSEN, Anders**
 **4000 Roskilde (DK)**

(74) Representative: **Guardian**
 **IP Consulting I/S**
 **Diplomvej, Building 381**
 **2800 Kgs. Lyngby (DK)**

(56) References cited:
**EP-A1- 2 384 727     WO-A1-2013/135271**
**US-A- 5 168 503**

• **GAO JING ET AL: "Design and output performance simulation of a novel 561 nm medical yellow laser", LASER PHYSICS AND LASER TECHNOLOGIES (RCSLPLT) AND 2010 ACADEMIC SYMPOSIUM ON OPTOELECTRONICS TECHNOLOGY (ASOT), 2010 10TH RUSSIAN-CHINESE SYMPOSIUM ON, IEEE, PISCATAWAY, NJ, USA, 28 July 2010 (2010-07-28), pages 20-22, XP031784976, ISBN: 978-1-4244-5511-9**

## Description

### Field of the invention

The present invention relates to a medical laser system for ophthalmological surgery, such as photocoagulation.

### Background

Lasers are extensively used in the medical field for surgery, such as ophthalmological surgical systems, e.g. as described in US 2004/0078029.

For the laser sources, a number of technologies are known, including laser diodes, e.g. as described in US 4,917,486. Recently, optically pumped semiconductor lasers, e.g. as described in US 6,370,168, have been widely adopted by manufacturers of for example photocoagulation systems. Such systems are used at large medical centers and hospitals. In addition, various techniques are today being explored to automate the surgery procedures, see for example Becker, B. C., MacLachlan, R. A., Lobes, L. A. and Riviere, C. N. (2010), Semiautomated intraocular laser surgery using handheld instruments. Lasers Surg. Med., 42: 264-273. doi: 10.1002/lsm.20897. Other works are performed by Yang, S., Lobes, L. A., Martel, J. N. and Riviere, C. N. (2015), Handheld-automated microsurgical instrumentation for intraocular laser surgery. Lasers Surg. Med., 47: 658-668. doi: 10.1002/lsm.22383). Despite these efforts, deployment of ophthalmological surgical systems, including photocoagulation lasers, at point-of-care level is largely absent.

In this context it is generally desirable to provide a medical laser system with improved ease of use for the medical doctor. In particular, it is desirable to provide a system with improved accuracy and/or faster treatment time while ensuring safety and high quality of surgery.

It is further generally desirable to provide medical laser systems that can be manufactured in a cost-efficient manner.

It is further generally desirable to provide medical laser systems that are durable.

The ability to control various performance parameters of a laser system is essential for most laser systems. These performance parameters can include optical power, beam diameter, astigmatism, ellipticity, intensity noise, beam quality, wavelength, spectral line width, side mode suppression ratio, beam focusing, beam pointing direction or a combination thereof. Depending on the application, some of these should simply be stabilized whereas other parameters must be changeable at the request of the user or as a part of an automated procedure.

The operating parameters of the laser system may be controlled by a laser controller. The controllable parameters may e.g. include one or more injection currents of the laser source. In particular, the laser controller may be operable to adjust the controllable parameters to optimize one or more performance parameters of the laser system, e.g. one or more of the performance parameters mentioned above, e.g. with respect of the output radiation of the laser system.

For example, WO 2013/135271 discloses a method for controlling beam quality and stability of a laser apparatus that comprises a tapered diode laser and a frequency converter. This prior art method comprises control loops where the current to the ridge section, the current to the tapered section and the temperature of a tapered diode laser are all iteratively adjusted to optimize the beam quality and stability of the laser. The temperature of the frequency converter may then be adjusted so as to optimize the efficiency of the frequency conversion process.

One performance parameter of particular interest in the context of ophthalmological surgery is the optical power of the output of the laser system. It is typically desirable that the optical output power can be selected by the user in order to allow the user to adjust the output power to the requirements of the individual surgical procedure. It is thus generally desirable to provide a laser system that allows setting the optical output power within a wide range. Preferably, the laser system should include a control mechanism for adjusting the optical output power of the laser system in a reliable manner and without unduly affecting other performance parameters of the output beam, such as one or more of the following: beam quality, astigmatism, wavelength.

While an attenuator may be used to attenuate the source beam before it is fed to the frequency converter and, hence adjust the power of the frequency-converted output beam, this would involve an additional component and increase the manufacturing costs of the system.

Accordingly, there is a need for an improved method for adjusting the optical output power of a laser system for opthalmological surgery.

### Summary

According to a first aspect, disclosed herein is a medical laser system for ophthalmological surgery, such as photocoagulation or another method of photo-thermal stimulation, the laser system comprising:

- a laser device configured to emit laser radiation at a visible wavelength; and

- a laser controller configured to control the laser system;

wherein the laser device comprises:

- a laser source configured to emit a source radiation and

- a frequency converter configured to receive the source radiation and to output said emitted laser radiation as a frequency-converted output of a frequency conversion process, the emitted laser radiation having an optical output power.

The laser controller is configured to control the optical output power of the emitted laser radiation by adjusting an operating temperature of the laser source and/or an operating temperature of the frequency converter.

The frequency conversion process is a phase-sensitive process which usually requires phase matching to be efficient. For example, a typical example of frequency conversion is frequency doubling, e.g. by second harmonic generation by a frequency converter in the form of a non-linear crystal. In such a process, phase matching causes the second-harmonic field contributions generated at different locations in the nonlinear crystal to interfere constructively at the crystal's exit face. Generally, the efficiency of the frequency conversion process may be measured as the ratio of the optical power of the output frequency converted radiation relative to the optical power of the input source radiation.

With suitable phase matching and a source beam of high intensity, high beam quality, and low to moderate optical bandwidth, achievable power conversion efficiencies often exceed 50%, sometimes even 80% or more. On the other hand, the conversion efficiencies are typically extremely small when phase matching does not occur.

Generally, the efficiency of the frequency conversion process depends on the wavelength of the radiation being converted. The functional dependence of the efficiency on the wavelength depends on the operating temperature of the frequency converter. In particular, the conversion efficiency has a maximum at an optimum wavelength where optimal phase matching occurs. The location of the maximum (i.e. the optimum wavelength) depends on the operating temperature of the frequency converter, since the phase matching condition depends on the operating temperature of the frequency converter.

In embodiments of the laser system described herein, the laser controller is configured to adjust the operating temperature of the laser source and/or the operating temperature of the frequency converter until the efficiency of the frequency conversion process at the wavelength of the source radiation is smaller than a maximum of the efficiency of the frequency converter as a function of wavelength and, in particular smaller than a maximum of the efficiency of the frequency converter as a function of wavelength at the operating temperature of the frequency converter resulting from the adjustment of the operating temperature of the laser source and/or the operating temperature of the frequency converter. Hence, the laser controller is configured to adjust the operating temperature of the laser source and/or the operating temperature of the frequency converter until the wavelength of the source radiation is different from the optimum wavelength of the frequency conversion process.

As an example, the laser system may perform the frequency conversion in a 40 mm long crystal of periodically poled lithium niobate operate and be able to operate at one of two operating points; one where the wavelength is detuned 10 pm from the maximum (or, correspondingly, a detuning of the crystal temperature by 0.1 K), corresponding to an efficiency of about 95% of the maximum value and one where the wavelength is detuned 35 pm from the maximum (or, equivalently, a detuning of the crystal temperature of 0.4 K), corresponding to an efficiency of 50% of the maximum value. In some embodiments, such adjustment may corresponds to wavelength shifts of 10-100 pm from the optimal wavelength, corresponding to crystal temperature shifts on the order of 0.1 - 1 K.

Hence, the process adjusts the operating temperature of the laser source and/or the operating temperature of the frequency converter so as to operate the frequency converter in a non-optimized (i.e. non-phase-matched) regime, i.e. where the frequency converter is operated at an efficiency below the maximum efficiency (for the operating temperature of the frequency converter resulting from the adjustment) and where the optical output power is below a maximum optical output power that may otherwise be achieved when the frequency converter is operated at maximum efficiency.

The inventors have realized that the optical output power of the emitted laser radiation may efficiently be controlled by adjusting the operating temperature of the laser source and/or by adjusting the operating temperature of the frequency converter. This adjustment allows the optical output power of the emitted laser radiation to be controlled without the need for a separate attenuator, thus reducing manufacturing costs. Moreover, certain performance parameters of many types of laser sources can be controlled by adjusting one or more control parameters of the laser source, such as an injection current. While these control parameters may also affect the optical output power of the emitted laser beam, the inventors have realized, that the use of the operating temperature of the laser source and/or of the frequency converter allows setting the optical output power without unduly affecting other performance parameters of the laser source.

Accordingly, the process allows the optical output power to be controlled to a selected target value that is smaller than the maximum obtainable optical output power of the laser system, e.g. a target value below 95% of the maximum obtainable optical output power, e.g. a target value below 90% of the maximum obtainable optical output power, e.g. a target value below 80% of the maximum obtainable optical output power, e.g. a target value below 70% of the maximum obtainable optical output power, e.g. a target value below 60% of the maximum obtainable optical output power, e.g. a target value below 50% of the maximum obtainable optical output power, e.g. a target value below 25% of the maximum obtainable optical output power. The upper limit of the sustainable target value in this type of control may be determined by small instabilities and fluctuations in the laser diode. If the power set point is set too high and the laser diode experiences brief fluctuations, the maximum obtainable optical output power may briefly drop below the target value and the power lock is lost. A re-initialisation of the temperature feedback loop may thus be necessary to re-stabilise the power. The lower limit may be determined by the shape of the phase matching curve far from the peak, which is less well defined and, especially in the case of periodically poled crystals, strongly affected by irregularities in the periodically reversed crystal domains that result from the poling process. A typical value of a sustainable upper limit is 95% and a typical value of a practical lower limit is 2%.

In some embodiments, the laser controller is configured to receive a user input indicative of the target optical output power, i.e. the user may select the desired optical output power, e.g. between a minimum optical output power (which may be larger or equal zero) and a maximum obtainable optical output power. Responsive to the user input, the laser controller adjusts the operating temperature of the laser source and/or the operating temperature of the frequency converter until the optical output power of the emitted laser beam is at the target value, or at least within a predetermined tolerance margin around the target value. As described here, the laser controller actively adjusts the operating temperature of the laser source and/or the operating temperature of the frequency converter so as to operate the frequency converter in a non-phase matched state, i.e. away from the optimised phase-matched state.

In some embodiments, the laser controller is configured to perform the adjustment of the optical output power while maintaining other control parameters of the laser source, in particular the injection current(s) to the laser source, constant. Hence, in some embodiments, the adjustment of the optical output power is performed without adjusting the injection current(s) of the laser source. For example, the laser controller may be configured to perform an initial adjustment of one or more control parameters of the laser source, e.g. an injection current, an operating temperature of the laser source, and/or the like, so as to optimize one or more performance parameters of the laser source and or the laser system, such as beam quality, stability, astigmatism, maximum output power, etc., i.e. to bring the laser source into an optimised operational state. Once optimised, one or more of the control parameters (e.g. the injection currents) may subsequently be kept constant or only be used to maintain stable performance parameters. The subsequent adjustment of the optical output power to a user-selected target value may be done by (preferably only) adjusting the operational temperature of the frequency converter and/or the operational temperature of the laser source without adjusting the injection current(s) of the laser source, or at least without adjusting the injection current(s) other than in order to maintain stable operation of the laser source, preferably at the optimised operational state. In some embodiments, the subsequent adjustment of the optical output power to a user-selected target value may be done by (preferably only) adjusting the operational temperature of the frequency converter without adjusting the injection current(s) or the operational temperature of the laser source, or at least without adjusting the injection current(s) or operational temperature of the laser source other than in order to maintain stable operation of the laser source, preferably at the optimised operational state.

The inventors have realized that it may be sufficient to only adjust the operating temperature of the laser source and/or the operating temperature of the frequency converter so as to operate the frequency converter in a non-optimized regime in order to adjust the optical output power of the laser system to a selected target value within an operating range of the laser system. Hence, embodiments of the laser system provide a particularly simple control mechanism. Moreover, the optical output power may be selected in a wide operating range without significantly altering the operational conditions of the laser source and, hence, while maintaining stable operation of the laser source, preferably in an optimized operational state. In some embodiments the operating range may be between 50 mW and 2W, such as between 100 mw and 1W.

In some embodiments, the laser controller is configured to adjust the operating temperature of the frequency converter, such as configured to adjust only the operating temperature of the frequency converter until the optical output power of the emitted laser beam is at the target value. Using the operating temperature of the frequency converter as a control parameter for adjusting the optical output power of the laser system reduces the influence of the power adjustment on the performance parameters of the laser source - and hence on the beam properties - even further.

It will be appreciated that, after having set the optical output power of the emitted radiation to the selected value, minor variations - e.g. of up to 2%, such as up to 1% of the selected target value - of the optical output power may be performed by adjusting an injection current of the laser source, e.g. to stabilize and/or fine tune the operational state of the laser system at the new target value of the optical output power, and without bringing the frequency converter into a phase-matched regime (unless the selected target value is the maximum achievable output power which can only be achieved when phase matching occurs).

In some embodiments, the laser device is configured to emit radiation at a visible wavelength and having a

beam quality factor $M^2$ of less than 3, e.g. less than 2.5, e.g. less than 2, e.g. less than 1.5, e.g. less than 1.3. This may e.g. be achieved by selecting a suitable laser source, e.g. a tapered laser diode or a MOPA device and by suitably selecting and/or controlling the injection currents of the laser source and, optionally by selecting the operating temperature of the laser source.

The medical laser system is suitable for use by a medical doctor for performing ophthalmological surgery to a patient, such as a myopic patient. When the medical doctor performs surgery to the retina of a patient, where the doctor moves from one retina area to another in such a patient, it is desirable that the laser system can substantially maintain focus when the laser beam is directed towards different areas. When the laser device emits radiation having a beam quality factor $M^2$ of less than 3, the focal depth of the laser is increased and the medical doctor does not need to refocus the laser spot each time the spot is moved to another area of the retina. This provides an advantage in terms of ease-of-use to the medical doctor.

In the context of non-visible laser treatment it may otherwise be difficult, if not impossible, to detect when the beam moves out of focus. An out-of-focus, non-visible laser treatment may be non-therapeutic. Hence, in non-visible laser treatments embodiments of the laser system disclosed herein that are capable of maintaining focus when the laser beam is moved are particularly beneficial.

A further advantage of some embodiments of the laser system disclosed herein is a reduced risk of error - hence, a safer surgical operation. It is a further advantage that treatment time is reduced, as the medical doctor does not need to spend time on refocusing the laser system. For semi- or fully-automated systems, for example systems using automatically scanned patterns for treatment, the optics can be simplified and/or wider scan areas can be achieved with low $M^2$.

In particular, in some embodiments, the system further comprises:

- one or more focussing actuators for adjusting a focal point of the emitted laser radiation or of the output radiation;

- one or more direction actuators for adjusting a light-emitting part of the laser system so as to selectively direct the emitted radiation or the output radiation to different target areas at different distances from the light-emitting part; and

- a controller configured to control the medical laser system to

   a) emit a focussed beam towards a first target area; wherein the one or more focussing actuators are set so as to focus the focussed beam a first focal distance;

   b) adjust the one or more direction actuators so as to direct the focussed beam to a second target area, and to

   c) emit the focussed beam towards the second target area without refocussing the emitted radiation or the output radiation.

The $M^2$ parameter is a widely used measure of beam quality of a laser beam. The $M^2$ parameter may be measured as defined in the ISO/DIS 11146 standard that defines that $M^2$ should be calculated from a series of measurements by focusing the beam with a fixed position lens of known focal length, and then measuring the characteristics of the beam waist and divergence. In particular, $M^2$ may be measured by measuring the beam width through a focus. The $M^2$ parameter always has a value greater or equal than 1.

In some embodiments, the laser system further comprises a beam delivery system, e.g. comprising a light guide, configured to receive the emitted laser radiation and to direct an output radiation to a target area, e.g. biological tissue. In such embodiments, it may be advantageous that the output radiation has a beam quality factor $M^2$ of less than 3. The beam delivery system may further comprise a fiber focus assembly and/or a slit lamp adapter.

In some embodiments, the laser system and, in particular, the laser source is a continuous-wave (CW) laser system. The laser system may comprise a user-controllable shutter operable to block the emitted laser beam from being directed to a target area. In particular, the laser controller may be operable to adjust the optical output power to a selected target value while the shutter is in a closed state. In some embodiments the laser controller is operable to prevent opening of the shutter during adjustment of the optical output power.

The frequency converter may be a frequency doubler, e.g. operable to perform second harmonic generation or sum-frequency conversion. In some embodiments, the medical laser system comprises an infrared laser source and a frequency converter operable for frequency doubling the infrared laser light to visible laser light. The frequency converter may be external to the laser cavity, thereby allowing control of the operating temperature of the frequency converter without affecting the operation of the laser cavity. The frequency converter may be used in single pass or multipass configurations. In some embodiments, the frequency converter is a nonlinear crystal. Preferably, the laser source, e.g. the infrared laser source, and/or the visible laser light has a beam quality, $M^2$, of less than 3, such as less than 2, such as less than 1.5, such as less than 1.3.

According to one aspect, a method is provided for adjusting the optical output power of a laser system in a medical laser system to a selected target value, such as a system for ophthalmological surgery, such as a photocoagulation laser system or another photo-thermal stimulation laser system, the laser system comprising:

- a laser source emitting source radiation in a first wavelength interval, such as an infrared laser source emitting infrared light;

- a frequency converter having an input and an output, the frequency converter being configured to receive the source radiation from the laser source at the input and to convert the source radiation to converted radiation in a second wavelength interval, e.g. visible light, and the output being configured to output the converted radiation, and an optical output power of the converted radiation being dependent on a operating temperature of the frequency converter,

- a control element, e.g. a heater and/or a cooling element, operable to adjust and stabilize the operating temperature of the frequency converter,

- an output configured to allow the converted radiation to exit the laser system,

wherein the method comprises actively controlling the temperature of the frequency converter so as to adjust and/or stabilize the optical output power emitted by the laser system by performing the following steps:

a. determining the optical output power emitted by the laser system,

b. comparing the determined optical output power emitted by the laser system to the selected target value,

c. adjusting the operating temperature of the frequency converter based on the comparison in (b) so as to reduce the magnitude of the difference between the determined optical output power and the selected target value.

The target value may be user-selected, e.g. selected at the beginning or during a treatment procedure.

Nevertheless, in some embodiments, a different control mechanism may be employed, e.g. based on an adjustment of additional or alternative controllable parameters.

The optical output power may e.g. be determined by a photodetector receiving a portion of the optical output power of the emitted laser beam, e.g. by means of a beam splitter. The steps of the above method may be performed or at least controlled by a suitable laser controller (also referred to as a laser control unit), e.g. by an ASIC or a suitably programmed microprocessor. To this end, the laser system may comprise a photodetector operable to monitor the optical output power of the emitted laser radiation.

In some embodiments, the laser source - e.g. the infrared laser source - comprises a tapered diode laser, see for example "Tapered diode lasers at 976nm with 8W nearly diffraction limited output power", Kelemen, M T; Weber, J; Kaufel, G; Bihlmann, G; Moritz, R; et al. Electronics Letters 41.18 (Sep 1, 2005), or Jensen, O.B.; Hansen, A.K.; Muller, A.; Sumpf, B.; Unterhuber, A.; Drexler, W.; Petersen, P.M.; Andersen, P.E. "Power Scaling of Nonlinear Frequency Converted Tapered Diode Lasers for Biophotonics", Selected Topics in Quantum Electronics, IEEE Journal of, On page(s): 307 - 321 Volume: 20, Issue: 2, March-April 2014, or Sumpf, B.; Hasler, K.-H.; Adamiec, P.; Bugge, F.; Dittmar, F.; Fricke, J.; Wenzel, H.; Zorn, M.; Erbert, G.; Trankle, G. "High-Brightness Quantum Well Tapered Lasers", Selected Topics in Quantum Electronics, IEEE Journal of, On page(s): 1009 - 1020 Volume: 15, Issue: 3, May-june 2009. While tapered diode lasers have been found to be a suitable laser source for a medical laser system described herein, the optical power of tapered diode lasers is difficult to control and, in particular, difficult to adjust without affecting other performance parameters of the laser source, such as beam quality, astigmatism, stability etc. Accordingly, adjusting the optical output power of the laser system by controlling the operating temperature of the frequency converter and/or the laser source has been found to be particularly useful, as adjustments of the injection currents (the ridge current and the tapered current) of the tapered diode laser in order to adjust the output power the the laser system to a new target value /setpoint can be avoided.

In alternative embodiments, the laser source - such as an infrared laser source-comprises a master oscillator (MO) and a power amplifier (PA) in a hybrid configuration, i.e. with the MO and the PA being provided as separate units. The hybrid MO and PA configuration may comprise an isolator positioned in a beam path between the MO and the PA. The hybrid MO and PA configuration may comprise a tapered PA. The MO may be a DFB laser.

In some embodiments, the medical laser system comprises a single-mode (in particular a single axial mode), and/or single-frequency, diode laser providing infrared radiation having a suitable average power. The average power of the diode laser may be chosen based on e.g. one or more of the following factors: The desired overall output of the laser apparatus, the gain of the subsequent amplification section (often, a low gain may be desirable), the power level that can be handled by the subsequent isolator, the sensitivity of the system to feedback. For example, generally a higher power is desirable, but the power should be balanced against the capabilities of the isolator and based on effects of possible reflections. In some embodiments, the diode laser has an average power of less than 10 mW. In other embodiments, the diode laser has an average power of more than 10 mW, such as more than 20 mW, such as more than 30 mW, such as more than 40 mW, and less than 200 mW, such as less than 150 mW, such as less than 100 mW, such as less than 50 mW. In other embodiments, the diode laser has an average power between 30 - 100 mW. In some embodiments, the medical laser system comprises an isolator unit being configured to receive the infrared radiation and isolate the single-mode diode laser from optical feedback. In some embodiments, the medical laser system comprises a tapered diode amplifier configured to receive the infrared radiation having passed through the isolator and amplify the radiation to an average power level of 100 mW or more, such as 1W or more, such as 3 W or more, such as 4 W or more, such as 5 W or more, e.g. between 3 W and 5 W, or between 5 W and 6 W, or even 6 W or more. In some embodiments, the medical laser system comprises a single-pass frequency conversion units being configured to receive the amplified infrared radiation and convert it to visible radiation. The optical output power of the visible radiation may e.g. be larger than 0.5 W, such as 1 W or more. In embodiments where the laser source comprises a MOPA, in order to design the MOPA for highest robustness against optical feedback, the optical power out of the single-mode diode laser should be higher than the so-called saturation power. The saturation power may generally be defined as the optical power of an input signal which in the steady state leads to a reduction in the gain to half of its small-signal value, i.e. corresponding to 3dB. Once the input power to the amplifier is higher than the saturation power, the gain in the tapered amplifier is "bleached" by the input. Effectively this means that the input power is high enough to extract substantially all possible gain out of the amplifier. In such configuration, light travelling in the opposite direction (optical feedback) will experience less gain from the amplifier and will thereby remain limited in power. Accordingly, in some embodiments, the laser device comprises:

- a master oscillator configured to emit a master radiation having a first optical power;
- an optical amplifier configured to receive and amplify the master radiation and to output the amplified radiation as the source radiation, the optical amplifier defining a saturation power; wherein the first optical power is larger than the saturation power of the optical amplifier; and
- optionally, an optical isolator positioned in a radiation path of the master radiation between the master oscillator and the optical amplifier.

In some embodiments, the medical laser system comprises an anamorphic lens to collimate infrared light from the output of a tapered PA. The advantage of this is that fewer components are needed and a compact design is facilitated.

In some embodiments, the medical laser system comprises an anamorphic lens to correct for astigmatism of infrared light from the PA.

In particular, in some embodiments, the laser system comprises a laser device configured emit radiation at a visible wavelength; wherein the laser source comprises:

- a substrate;

- a master oscillator mounted on the substrate and configured to emit a master radiation;

- an optical amplifier mounted on the substrate and configured to receive and amplify the master radiation and to output the amplified radiation as the source radiation;

- an optical isolator mounted on the substrate in a radiation path of the master radiation between the master oscillator and the optical amplifier;

- one or more optical elements mounted on the substrate and configured to direct the master radiation from the master oscillator into the optical isolator and/or to direct the master radiation from the optical isolator towards the optical amplifier; wherein the one or more optical elements are mounted on the substrate by one or more deformable mountings.

Generally, in some embodiments, the medical laser system comprises deformable metal mounts for one or more optical elements. In particular, the mounts may be repeatedly "reworkable" and are by their design fixed when not moved by external activation.

In some embodiments, at least the laser source is packaged in an oxygen atmosphere with a predetermined oxygen concentration. The present inventors have realized that if high power lasers (laser with power levels above 0.5W, such as above 1W, such as above 3W, such as above 5W, such as above 10W) in the 1000 nm range are packaged in an inert atmosphere such as Nitrogen or Nitrogen and Helium mixtures as is standard in semiconductor optical packaging residual hydrocarbons even at very low levels can cause so-called PIF (Packaging Induced Failure) of lasers at the facet. This happens because at very high optical intensities at the laser facets there are fourth order optical conversion which produces photons with enough energy to crack the hydrocarbons. A layer of amorphous carbon will grow on the facet changing first the reflectivity and eventually the absorption at the facet. Eventually the absorption leads to heating of the facet and ultimately melting and destruction. The present inventors have realized that this can be avoided by packaging, not in an inert atmosphere, but in dilute oxygen where the carbon is converted to carbon dioxide at the facet. A getter for water is required in such packages because residual hydrogen will combine with the oxygen to produce water which ultimately can lead to corrosion in the package if not containing a getter.

In a preferred embodiment, the medical laser system comprises a hermetically sealed package containing a tapered diode laser, and/or a hybrid MO and PA configuration.

In some embodiments, the emitted laser light has an intensity and wavelength suitable for therapeutic treatment. In particular, the emitted laser radiation may have a wavelength in the range from 480 nm to 632 nm, such as from 530 nm to 580 nm, such as around 532 nm, around 561 nm or around 577 nm.

In some embodiments, the medical laser system comprises a low-power laser beam (e.g. having an average power less than 1 mW) to serve as pilot beam for the medical doctor.

## Brief description of the drawings

FIGs. 1 - 2 show schematic block diagrams of examples of a laser device.

FIG. 3 illustrates the adjustment of the optical output power by adjusting the operating temperature of the frequency converter.

FIG. 4 shows a schematic block diagram of a medical laser system.

FIG. 5 shows a schematic block diagram of a scanning medical laser system.

## Detailed description

Various aspects and embodiments of a medical laser apparatus disclosed herein will now be described with reference to the drawings.

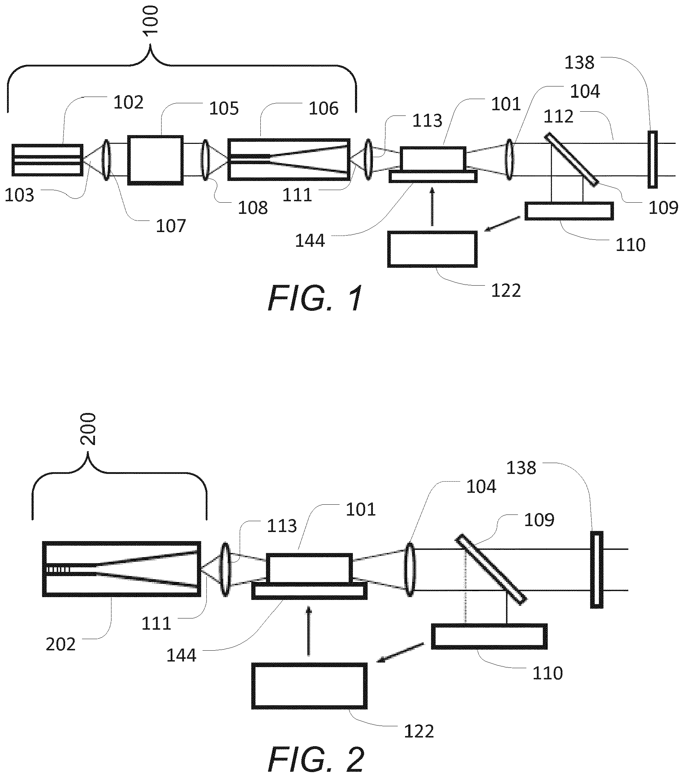

FIG. 1 shows an example of a laser device comprising a laser source 100 and a frequency converter 101, such as a frequency doubling crystal. The laser source is provided in a configuration where the radiation 103 from a laser 102 is coupled through an isolator 105 to a semiconductor optical amplifier 106. This configuration is referred to as a hybrid master oscillator power amplifier (MOPA) with isolation stage between the laser oscillator and the power amplifier. The laser 102 is also referred to as the master oscillator (MO); it may e.g. be a distributed feedback (DFB) laser or another suitable single-frequency laser. The power amplifier (PA) 106 may e.g. be a tapered optical amplifier. The amplified beam 111 is then fed into the frequency converter 101 which outputs the output beam 112 of the laser device as a frequency-converted beam. The laser device comprises a temperature control device 144, e.g. a Peltier element, a resistor or the like, operable to control the temperature of the frequency converter 101. To this end, the laser device comprises a laser control unit 122 operable to control the temperature control device. It will be appreciated that the laser control unit may also control other control parameters of the laser device.

The isolator is a bulk optic component utilizing a free space propagating collimated optical beam. To this end, the laser source comprises a collimating lens 107 arranged in the beam path between the laser 102 and the isolator 105 and configured to collimate the radiation from the laser and direct it into the isolator 105. The laser source further comprises an optical lens 108, e.g. a collimating lens, arranged in the beam path between the isolator and the optical amplifier 106 and configured to focus the collimated beam from the isolator into the optical amplifier. Advantages of this configuration are that the isolator loss can be compensated by gain in the power amplifier. Thus in the 1000 nm wavelength region low cost miniature thin film garnet isolators can be used. This reduces overall cost, improves performance, and reduces overall size relative to an integrated MOPA or high powered tapered laser followed by a larger-aperture low-loss isolator while maintaining or improving insensitivity to reflection.

In this hybrid configuration a proper alignment between the laser and the collimating lens and between the collimating lens and the power amplifier is critical. In one embodiment, this alignment is achieved by deformable lens holder alignment structures. In particular all components are either directly or indirectly mounted on a substrate. The lenses 107 and 108 may be mounted on the substrate via the deformable lens holder alignment structures, also referred to as deformable lens mounts. The deformable lens holder alignment structures may e.g. be structures as described in US06559464.

The laser device further comprises a photodetector 110 or other suitable sensor operable to monitor the optical output power of the emitted, frequency-converted laser beam 112. To this end, the laser device comprises a collimating lens 104 collimating the emitted laser beam 112 and a beam splitter 109 or other suitable optical element for redirecting a minor portion of the collimated emitted laser beam 112 towards the photodetector 110. It will be appreciated that the laser device may further comprise one or more additional detectors for monitoring performance characteristics of the master oscillator, e.g. for monitoring the wavelength or output power. The output of the photodetector is fed into the laser control unit 122 so as to allow the laser control unit to control the temperature control device 144 based on the optical power monitored by the photodetector and based on a selected target value of the output power which may be user-selected. The laser device further comprises a shutter 138 for selectively blocking the emitted laser beam 112. The shutter may be user-controlled but the laser control unit may prevent the shutter from opening when the power (or other performance parameters of the laser device) is/are adjusted.

The laser device further comprises a lens assembly 113 positioned in the beam path between the optical amplifier of the laser source and the frequency converter. The lens assembly may consist of a single anamorphic lens or of a set of lenses configured so as to compensate for astigmatism in the output radiation from the tapered amplifier. The lens assembly may also be mounted on one or more deformable mounts as described in connection with lenses 107 and 108.

It is known that high power semiconductor lasers in the 1000nm wavelength range packaged in hermetic environments can suffer sudden degradation due to breakdown of residual contaminates at the facet component. In the hybrid configuration shown in the above examples not only could his happen at the laser facet but at both facets of the optical amplifier. This degradation can be avoided by packaging the laser in an atmosphere containing a slight amount of oxygen.

FIG. 2 shows another example of a laser device comprising a laser source 200 and a frequency converter 101, such as a frequency doubling crystal. The laser device of FIG. 2 is similar to the one of FIG. 1 except that the laser source 200 of the laser device of FIG. 2 is a tapered laser diode 202.

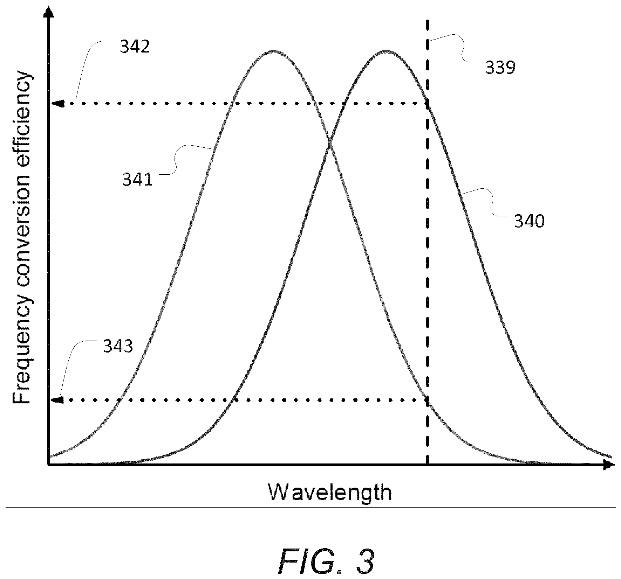

FIG. 3 illustrates the adjustment of the optical output power by adjusting the operating temperature of the frequency converter. In particular, FIG. 3 illustrates the dependence of the frequency conversion efficiency on the operating temperature of the frequency converter and on the wavelength. FIG. 3 shows the efficiency of the frequency conversion process as a function of wavelength for two different operating temperatures. The conversion efficiency as a function of wavelength for a first operating temperature is schematically illustrated by curve 340, while the conversion efficiency as a function of wavelength for a second, different operating temperature is schematically illustrated by curve 341. As can be seen from FIG. 3, a change in operating temperature of the frequency converter results in a shift of the maximum efficiency to a different wavelength. The frequency of the source radiation emitted by the laser source, i.e. the wavelength before frequency conversion, is illustrated by the dashed line 339. It will be appreciated that the source radiation in practice will be a narrow peak of finite width, i.e. the line 339 may be considered as representing the center wavelength of the source radiation.

As can easily be seen from FIG. 3, for different operating temperatures of the frequency converter, the conversion efficiency at the actual source wavelength differs considerably, as illustrated by the dotted lines 342 and 343, respectively.

Accordingly, by varying the operating temperature of the frequency converter, the conversion efficiency at which the frequency converter converts the source radiation can be controlled. Since, for a given optical power of the source radiation, the optical power of the frequency-converted output beam depends on the conversion efficiency, the optical output power can be controlled by adjusting the operating temperature of the frequency converter.

It will further be noted that the wavelength of the source radiation depends on the operating temperature of the laser source. Therefore, the power control may also be performed by adjusting the operating temperature of the laser source, which causes the dashed line to be moved along the horizontal axis relative to the conversion efficiency curve. However, controlling of the output power by adjusting the operating temperature of the laser source thus also changes the wavelength of the emitted, frequency-converted output beam which, in some embodiments may be less desirable.

Generally, the laser controller adjusts the operating temperature of the frequency converter and/or the operating temperature of the laser source such that the frequency converter operates at a wavelength on an upward or downward slope of the conversion efficiency as a function of wavelength and, in particular at a wavelength displaced from an optimum wavelength where that conversion efficiency has a maximum and/or displaced from a flat portion where the conversion efficiency substantially does not change in dependence of the wavelength. Considering that thermal adjustment of either a frequency converting crystal or of the laser diode itself is often no faster than a time scale of 1 s, a fast response in this stabilization and control scheme may be achieved when operating in a regime in which the slope of the phase matching curve is 10% of the maximum conversion efficiency per kelvin or more, such as 15% of the maximum conversion efficiency per kelvin or more.

The control process implemented by the laser control unit may utilise any suitable control loop, e.g. based on a monitored optical output power. An example of an algorithm for such a control loop may be based on the PID (proportional-integral-derivative) control loop scheme. In such an implementation, the desired output power (target value) as a function of time $t$ is denoted $P_s(t)$ and the measured output power is denoted P(t). The error $e(t) = P_s(t) - P(t)$ is the difference of the two. The temperature set point $T_s(t)$ of the frequency converter is the control variable. It can calculated as

$$T_s(t) = T_s(0) + K_P e(t) + K_I \int_0^t e(\tau)d\tau + K_D \frac{de(t)}{dt}$$

where $K_P$, $K_I$ and $K_D$ are constants that determine the temporal behavior of the control loop and $T_s(0)$ is an initial guess of the desired temperature set point, e.g. a predetermined constant. As time passes, the temperature set point is continually adjusted to be closer and closer to the value that results in the desired output power. If the desired output power is changed to a new value, e.g. due to a user input, the above control loop algorithm will subsequently change the temperature set point to achieve the new desired output power.

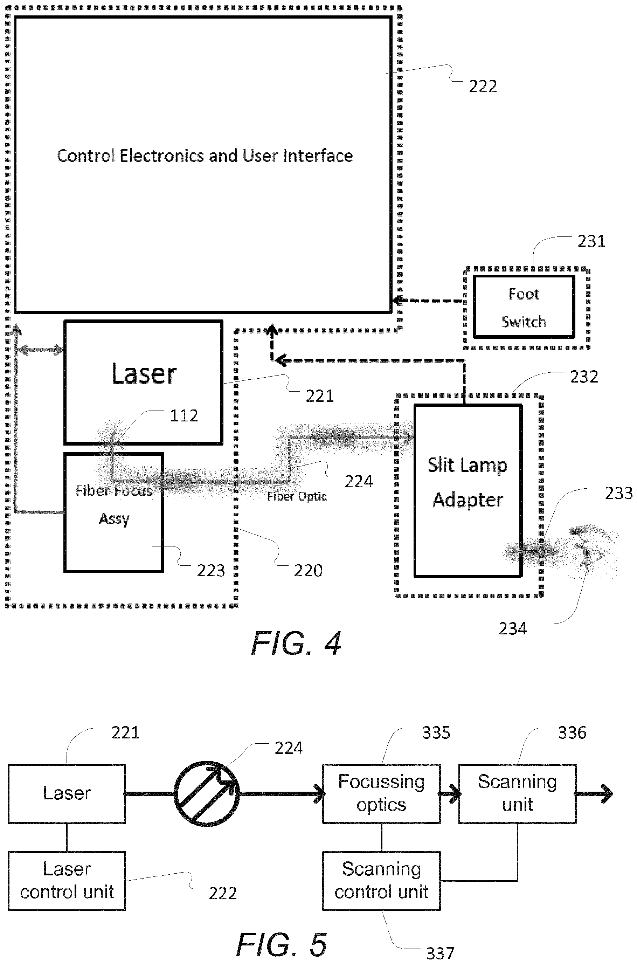

FIG. 4 shows a schematic block diagram of a medical laser system. The laser system comprises a laser unit 220, a foot switch 231 and a slit lamp adapter 232.

The laser unit comprises a laser device 221, a control unit 222 comprising control electronics and a user interface, and a fiber focus assembly 223.

The laser device 221 may be or comprise a laser device as described in FIG. 1 or 2. The laser device 221 is communicatively connected to the control unit 222, and the laser device outputs a laser beam 112 that is fed into the fiber focus assembly 223.

The fiber focus assembly 223 feeds the laser beam into an optical fiber 224 which is operable to guide the laser beam into the slit lamp adapter. The fiber focus assembly is also communicatively connected to the control unit 222. The fiber focus assembly comprises optics operable to focus the laser beam 112 into the optical fiber 224. The laser beam 112 is for use as a treatment beam. The fiber focus assembly may further be operable to couple a red (e.g. 635nm) diode "aiming beam" down the same optical path as the treatment laser. The fiber focus assembly may further house a photodiode pickoff to monitor the laser output for calibration and safety.

The slit lamp adapter 232 directs the laser beam as treatment beam 233 into a subject's eye 234. The slit lamp adapter comprises an optical train operable to focus the laser output to a user-selectable spot sizes at a target tissue. The slit lamp adapter may further comprise a dichroic mirror operable to reflect the aiming and treatment laser beams towards the target, but to allow other visible wavelengths to pass. The slit lamp adapter may further comprise one or more eye-safety filters operable to protect a user from laser reflections through the visual axis. The slit lamp adapter may further comprise a mechanical mount operable to adapt to pre-existing diagnostic slit lamps. The slit lamp adapter may further comprise circuitry, e.g. a PCB, operable to communicate with the control unit 222, e.g. for facilitating spot size selection

The foot switch 231 may be a wired or a wireless device that may comprise redundant contacts for safety. The foot switch may be operable to be pressed by the user so as to cause the system to deliver laser light to the target tissue. To this end, the foot switch may be operable to control a shutter for selectively blocking the laser light.

The foot switch 231 and the slit lamp adapter are communicatively connected to the control unit 222.

The control unit 222 may comprise one or more of the following:

- power input and AC/DC conversion circuitry, e.g. implemented as one or more PCBs,

- control circuitry, e.g. implemented as one or more control PCBs, which may include on-board firmware to provide commands to all sub systems on laser control, sub-system control, safety monitoring, and laser pulse generation, temperature control.

- a user Interface which may be include knobs and a display, and/or a touchscreen interface. The user interface allows the user to select the desired parameters and provides the user with system status.

FIG. 5 shows a schematic block diagram of a scanning medical laser system, e.g. a laser scanning photocoagulator. The scanning medical laser system comprises a laser device 221 and a laser control unit 222, e.g. as described in connection with FIG. 4, the laser device 221 is operable to feed a laser beam into an optical fiber 224, e.g. via a fiber focus assembly (not shown) as described in connection with FIG. 4. The optical fiber feeds the laser beam into a focussing unit 335 and further into a scanning unit 336. The focussing unit comprises adjustable focussing optics adjustable by one or focussing more actuators. The adjustable focussing optics of the focussing unit are controlled by a scanning control unit 337 which may be a separate control unit or integrated into laser control unit 222. For example, the focussing unit may be implemented as a slit lamp adapter, e.g. as described in connection with FIG. 4. Similarly, the scanning unit is controlled by the scanning control unit to direct the laser beam to desired target locations. The scanning unit 336 may e.g. comprise an X & Y axis galvanometer or other types of direction actuators which receives instructions from the control electronics 337 on the beam position and the pattern to be displayed. The blanking between each spot in a pattern may be provided by an electronic shutter, a mechanical shutter or by another galvanometer.

Generally, there are three axes in ophthalmic laser delivery that should be parfocal to get consistent and safe results. These are the visual, the illumination and laser axes must all be in focus at the same point. The illumination and visual axes are provided by the slit lamp and the laser is adapted to focus to their plane. Many lasers have a very narrow depth of focus which makes it difficult for the user to get consistent laser uptake because the laser is moving in and out of focus even though the visual and illumination fields are OK. The longer the laser depth of focus, the more forgiving the laser is to out of focus use and it makes it much easier on the Doctor to perform the treatment. The excellent beam quality of embodiments of the laser device described herein provides a longer depth of focus and causes the laser device to be much easier to use than prior art systems.

In the claims enumerating several means, several of these means can be embodied by one and the same element, component or item of hardware. The mere fact that certain measures are recited in mutually different dependent claims or described in different embodiments does not indicate that a combination of these measures cannot be used to advantage.

It should be emphasized that the term "comprises/comprising" when used in this specification is taken to specify the presence of stated features, elements, steps or components but does not preclude the presence or addition of one or more other features, elements, steps, components or groups thereof.

**Claims**

1. A medical laser system for ophthalmological surgery, such as photocoagulation or another method of photo-thermal stimulation, the laser system comprising:

   - a laser device configured to emit laser radiation at a visible wavelength; and
   - a laser controller configured to control the laser system;

   wherein the laser device comprises:

   - a laser source configured to emit a source radiation, and
   - a frequency converter configured to receive the source radiation and to output said emitted laser radiation as a frequency-converted output of a frequency conversion process, the frequency conversion process having an efficiency dependent on a wavelength of the source radiation, the efficiency having a maximum, the emitted laser radiation having an optical output power;

   wherein the laser controller is configured to adjust the optical output power of the emitted laser radiation to a selected target value by adjusting an operating temperature of the laser source and/or an operating temperature of the frequency converter such that the frequency converter is operated at an efficiency smaller than the maximum of the efficiency.

2. A medical laser system according to claim 1, wherein said radiation at a visible wavelength has a beam quality factor $M^2$ of less than 3.

3. A medical laser system according to any one of the preceding claims; further comprising:

   - one or more focussing actuators for adjusting a focal point of the emitted laser radiation or of the output radiation;
   - one or more direction actuators for adjusting a light-emitting part of the laser system so as to selectively direct the emitted radiation or the output radiation to different target areas at different distances from the light-emitting part; and
   - a controller configured to control the medical laser system to

     a) emit a focussed beam towards a first target area; wherein the one or more focussing actuators are set so as to focus the focussed beam a first focal distance;
     b) adjust the one or more direction actuators so as to direct the focussed beam to a second target area, and to

c) emit the focussed beam towards the second target area without refocussing the emitted radiation or the output radiation.

4. A medical laser system according to any one of the preceding claims; wherein the laser controller is configured to:

- determine an optical output power emitted by the laser system,
- compare the determined optical output power emitted by the laser system to a selected target value,
- adjust the operating temperature of the frequency converter based on the comparison of the determined optical output power emitted by the laser system with the selected target value so as to reduce the magnitude of the difference between the determined optical output power and the selected target value.

5. A medical laser system according to any one of the preceding claims; wherein the laser controller is further configured to control a performance parameter of the emitted laser radiation by controlling an injection current to the laser source.

6. A medical laser system according to any one of the preceding claims; wherein the laser device comprises a tapered diode laser.

7. A medical laser system according to any one claims 1 through 6; wherein the laser device comprises:

- a substrate;
- a master oscillator mounted on the substrate and configured to emit a master radiation;
- an optical amplifier mounted on the substrate and configured to receive and amplify the master radiation and to output the amplified radiation as the source radiation;
- an optical isolator mounted on the substrate in a radiation path of the master radiation between the master oscillator and the optical amplifier;
- one or more optical elements mounted on the substrate and configured to direct the master radiation from the master oscillator into the optical isolator and/or to direct the master radiation from the optical isolator towards the optical amplifier; wherein the one or more optical elements are mounted on the substrate by one or more deformable mountings.

8. A medical laser system according to any one of the preceding claims; comprising a sensor for detecting the optical output power of the emitted laser radiation; and wherein the laser controller is configured to adjust the operating temperature of the frequency converter and/or of the laser source until the detected optical output power matches the selected target value.

9. A medical laser system according to any one of the preceding claims; comprising a user-controllable shutter for selectively blocking the emitted laser radiation; and wherein the laser controller is configured to prevent opening of the shutter during adjustment of the optical output power.

10. A medical laser system according to any one of the preceding claims; wherein the selected target value is a user-selectable target value selectable between an uppermost selectable target value being between larger than 500 mW and smaller than 2 W; and wherein the lowermost selectable target value is between larger than 50 mW and smaller than 500 mW.

11. A medical laser system according to any one of the preceding claims; wherein the laser controller is configured to adjust the operating temperature of the frequency converter and/or of the laser source such that the frequency converter is operated in a regime in which a change of the efficiency of the conversion process as a function of the operating temperature of the frequency converter is at least 10% per Kelvin.

12. A medical laser system according to any one the preceding claims; wherein the laser controller is configured to adjust the operating temperature of the frequency converter and/or of the laser source such that the frequency converter is operated in a regime in which the efficiency of the conversion process is greater than 2% and less than 95% of a maximum conversion efficiency as a function of the operating temperature of the frequency converter and/or of the laser source.

13. A medical laser system according to any one of the preceding claims; wherein the laser controller is configured to adjust the operating temperature of the frequency converter and/or of the laser source until the wavelength of the source radiation is displaced from a wavelength at which maximum conversion efficiency is obtained by more than 10 pm and less than 100 pm, such as by more than 20 pm and less than 100 pm.

14. A medical laser system according to any one of the preceding claims; wherein the laser controller is configured to adjust the operating temperature of the frequency converter and/or of the laser source until the operating temperature of the frequency converter deviates from a temperature at which maximum conversion efficiency is obtained by more than 0.1 K and less than 1 K.

15. A method for adjusting the optical output power of a laser system in a medical laser system to a selected target value, such as a system for ophthalmological surgery, such as a photocoagulation laser system or another photo-thermal stimulation laser system, the laser system comprising:

- a laser source emitting source radiation in a first wavelength interval, such as an infrared laser source emitting infrared light;
- a frequency converter having an input and an output, the frequency converter being configured to receive the source radiation from the laser source at the input and to convert the source radiation to converted radiation in a second wavelength interval, e.g. visible light, and the output being configured to output the converted radiation, and an optical output power of the converted radiation being dependent on a operating temperature of the frequency converter,
- a control element, e.g. a heater and/or a cooling element, operable to adjustment and stabilize the operating temperature of the frequency converter,
- an output configured to allow the converted radiation to exit the laser system,

wherein the method comprises actively controlling the temperature of the frequency converter so as to adjust or stabilize the optical output power emitted by the laser system by performing the following steps:

a. determining the optical output power emitted by the laser system,
b. comparing the determined optical output power emitted by the laser system to the selected target value,
c. adjusting the operating temperature of the frequency converter based on the comparison in (b) so as to reduce the magnitude of the difference between the determined optical output power and the selected target value.

## Patentansprüche

1. Medizinisches Lasersystem für ophthalmologische Chirurgie, wie Photokoagulation oder ein anderes Verfahren zur photothermischen Stimulation, wobei das Lasersystem umfasst:

- eine Laservorrichtung, die konfiguriert ist, um Laserstrahlung bei einer sichtbaren Wellenlänge zu emittieren; und
- eine Lasersteuerung, die konfiguriert ist, um das Lasersystem zu steuern;

wobei die Laservorrichtung umfasst:

- eine Laserquelle, die konfiguriert ist, um eine Quellstrahlung zu emittieren, und
- einen Frequenzwandler, der konfiguriert ist, um die Quellstrahlung zu empfangen und um die emittierte Laserstrahlung als einen frequenzumgewandelten Ausgang eines Frequenzumwandlungsprozesses auszugeben, wobei der Frequenzumwandlungsprozess eine Effizienz aufweist, die von der Wellenlänge der Quellstrahlung abhängt, wobei die Effizienz ein Maximum aufweist, wobei die emittierte Laserstrahlung eine optische Ausgangsleistung aufweist;

wobei die Lasersteuerung konfiguriert ist, um die optische Ausgangsleistung der emittierten Laserstrahlung durch ein Einstellen einer Betriebstemperatur der Laserquelle und/oder einer Betriebstemperatur des Frequenzwandlers auf einen ausgewählten Zielwert einzustellen, sodass der Frequenzwandler bei einer Effizienz betrieben wird, die kleiner als das Maximum der Effizienz ist.

2. Medizinisches Lasersystem nach Anspruch 1, wobei die Strahlung bei einer sichtbaren Wellenlänge einen Strahlqualitätsfaktor $M^2$ von weniger als 3 aufweist.

3. Medizinisches Lasersystem nach einem der vorstehenden Ansprüche; weiter umfassend:

- ein oder mehrere Fokussierungsstellglieder zum Einstellen eines Brennpunkts der emittierten Laserstrahlung oder der Ausgangsstrahlung;
- ein oder mehrere Richtungsstellglieder zum Einstellen eines Licht emittierenden Teils des Lasersystems, um die emittierte Strahlung oder die Ausgangsstrahlung selektiv auf verschiedene Zielbereiche in verschiedenen Abständen von dem lichtemittierenden Teil zu richten; und
- eine Steuerung, die konfiguriert ist, um das medizinische Lasersystem zu steuern, um

a) einen fokussierten Strahl in Richtung eines ersten Zielbereichs zu emittieren; wobei das eine oder die mehreren Fokussierungsstellglieder gesetzt sind, um den fokussierten Strahl für eine erste Brennweite fokussieren;
b) das eine oder die mehreren Richtungsstellglieder einzustellen, um den fokussierten Strahl auf einen zweiten Zielbereich zu richten, und um
c) den fokussierten Strahl in Richtung des zweiten Zielbereichs zu emittieren, ohne die emittierte Strahlung oder die Ausgangsstrahlung erneut zu fokussieren.

4. Medizinisches Lasersystem nach einem der vorstehenden Ansprüche; wobei die Lasersteuerung konfiguriert ist, um:

- eine von dem Lasersystem emittierte optische Ausgangsleistung zu bestimmen,
- die von dem Lasersystem emittierte bestimmte optische Ausgangsleistung mit einem ausgewählten Zielwert zu vergleichen,
- die Betriebstemperatur des Frequenzwandlers basierend auf dem Vergleich der von dem Lasersystem emittierten bestimmten optischen Ausgangsleistung mit dem ausgewählten Zielwert einzustellen, um die Größe der Differenz zwischen der bestimmten optischen Ausgangsleistung und dem ausgewählten Zielwert zu verringern.

5. Medizinisches Lasersystem nach einem der vorstehenden Ansprüche; wobei die Lasersteuerung weiter konfiguriert ist, um einen Leistungsparameter der emittierten Laserstrahlung durch Steuern eines Injektionsstroms zu der Laserquelle zu steuern.

6. Medizinisches Lasersystem nach einem der vorstehenden Ansprüche; wobei die Laservorrichtung einen sich verjüngenden Diodenlaser umfasst.

7. Medizinisches Lasersystem nach einem der Ansprüche 1 bis 6; wobei die Laservorrichtung umfasst:

- ein Substrat;
- einen Hauptoszillator, der auf dem Substrat montiert ist und konfiguriert ist, um eine Hauptstrahlung zu emittieren;
- einen optischen Verstärker, der auf dem Substrat montiert ist und konfiguriert ist, um die Hauptstrahlung zu empfangen und zu verstärken und um die verstärkte Strahlung als die Quellstrahlung auszugeben;
- einen optischen Isolator, der auf dem Substrat in einem Strahlungspfad der Hauptstrahlung zwischen dem Hauptoszillator und dem optischen Verstärker montiert ist;
- ein oder mehrere optische Elemente, die auf dem Substrat montiert sind und konfiguriert sind, um die Hauptstrahlung von dem Hauptoszillator in den optischen Isolator zu richten und/oder um die Hauptstrahlung von dem optischen Isolator zu dem optischen Verstärker zu richten;

wobei das eine oder die mehreren optischen Elemente mit einer oder mehreren verformbaren Halterungen auf dem Substrat montiert sind.

8. Medizinisches Lasersystem nach einem der vorstehenden Ansprüche; umfassend einen Sensor zum Erfassen der optischen Ausgangsleistung der emittierten Laserstrahlung; und
wobei die Lasersteuerung konfiguriert ist, um die Betriebstemperatur des Frequenzwandlers und/oder der Laserquelle einzustellen, bis die erfasste optische Ausgangsleistung dem ausgewählten Zielwert entspricht.

9. Medizinisches Lasersystem nach einem der vorstehenden Ansprüche; umfassend einen von einem Benutzer steuerbaren Verschluss zum selektiven Blockieren der emittierten Laserstrahlung; und
wobei die Lasersteuerung konfiguriert ist, um ein Öffnen des Verschlusses während des Einstellens der optischen Ausgangsleistung zu verhindern.

10. Medizinisches Lasersystem nach einem der vorstehenden Ansprüche; wobei der ausgewählte Zielwert ein von einem Benutzer auswählbarer Zielwert ist, der zwischen einem obersten auswählbaren Zielwert, der zwischen größer als 500 mW und kleiner als 2 W liegt, auswählbar ist; und wobei der unterste auswählbare Zielwert zwischen größer als 50 mW und kleiner als 500 mW liegt.

11. Medizinisches Lasersystem nach einem der vorstehenden Ansprüche; wobei die Lasersteuerung konfiguriert ist, um die Betriebstemperatur des Frequenzwandlers und/oder der Laserquelle einzustellen, sodass der Frequenzwandler in einem Regime betrieben wird, in dem eine Änderung der Effizienz des Umwandlungsprozesses als eine Funktion der Betriebstemperatur des Frequenzwandlers zumindest 10% pro Kelvin beträgt.

12. Medizinisches Lasersystem nach einem der vorstehenden Ansprüche; wobei die Lasersteuerung konfiguriert ist, um die Betriebstemperatur des Frequenzwandlers und/oder der Laserquelle einzustellen, sodass der Frequenzwandler in einem Regime betrieben wird, in dem die Effizienz des Umwandlungsprozesses größer als 2 % und geringer als 95 % einer maximalen Umwandlungseffizienz als eine Funktion der Betriebstemperatur des Frequenzwandlers und/oder der Laserquelle ist.

13. Medizinisches Lasersystem nach einem der vorstehenden Ansprüche; wobei die Lasersteuerung konfiguriert ist, um die Betriebstemperatur des Frequenzwandlers und/oder der Laserquelle einzustellen, bis die Wellenlänge der Quellstrahlung von einer Wellenlänge verschoben ist, bei der die maximale Umwandlungseffizienz von mehr als 10 pm und weniger als 100 pm, wie von mehr als 20 pm und weniger als 100 pm, erhalten wird.

14. Medizinisches Lasersystem nach einem der vorstehenden Ansprüche; wobei die Lasersteuerung konfiguriert ist, um die Betriebstemperatur des Frequenzwandlers und/oder der Laserquelle einzustellen, bis die Betriebstemperatur des Frequenzwandlers von einer Temperatur abweicht, bei der eine maximale Umwandlungseffizienz von mehr als 0.1 K und weniger als 1 K erhalten wird.

15. Verfahren zum Einstellen der optischen Ausgangsleistung eines Lasersystems in einem medizinischen Lasersystem auf einen ausgewählten Zielwert, wie einem Lasersystem für ophthalmologische Chirurgie, wie ein Lasersystem für Photokoagulation oder ein anderes Lasersystem für photothermische Stimulation, wobei das Lasersystem umfasst:

    - eine Laserquelle, die eine Quellstrahlung in einem ersten Wellenlängenintervall emittiert, wie eine Infrarotlaserquelle, die Infrarotlicht emittiert;
    - einen Frequenzwandler, der einen Eingang und einen Ausgang aufweist, wobei der Frequenzwandler konfiguriert ist, um die Quellstrahlung von der Laserquelle an dem Eingang zu empfangen und um die Quellstrahlung in eine umgewandelte Strahlung in einem zweiten Wellenlängenintervall, z. B. sichtbares Licht, umzuwandeln, und wobei der Ausgang konfiguriert ist, um die umgewandelte Strahlung auszugeben, und wobei eine optische Ausgangsleistung der umgewandelten Strahlung von einer Betriebstemperatur des Frequenzwandlers abhängig ist,
    - ein Steuerelement, z.B. eine Heizvorrichtung und/oder ein Kühlelement, das betreibbar ist, um die Betriebstemperatur des Frequenzwandlers einzustellen und zu stabilisieren,
    - einen Ausgang, der konfiguriert ist, um zu ermöglichen, dass die umgewandelte Strahlung das Lasersystem verlassen kann,

    wobei das Verfahren ein aktives Steuern der Temperatur des Frequenzwandlers umfasst, um die optische Ausgangsleistung, die von dem Lasersystem emittiert wird, einzustellen und zu stabilisieren, indem die folgenden Schritte durchgeführt werden:

    a. Bestimmen der optischen Ausgangsleistung, die von dem Lasersystem emittiert wird,
    b. Vergleichen der bestimmten optischen Ausgangsleistung, die von dem Lasersystem emittiert wird, mit dem ausgewählten Zielwert,
    c. Einstellen der Betriebstemperatur des Frequenzwandlers basierend auf dem Vergleich in (b), um die Größe der Differenz zwischen der bestimmten optischen Ausgangsleistung und dem ausgewählten Zielwert zu verringern.

**Revendications**

1. Système laser médical pour chirurgie ophtalmologique, telle que la photo-coagulation ou un autre procédé de stimulation photo-thermique, le système laser comprenant :

- un dispositif laser configuré pour émettre un rayonnement laser à une longueur d'onde visible ; et
- un dispositif de commande de laser configuré pour commander le système laser;

dans lequel le dispositif laser comprend :

- une source laser configurée pour émettre un rayonnement source, et
- un convertisseur de fréquence configuré pour recevoir le rayonnement source et pour délivrer en sortie ledit rayonnement laser émis en tant que sortie convertie en fréquence d'un processus de conversion de fréquence, le processus de conversion de fréquence ayant une efficacité dépendant d'une longueur d'onde du rayonnement source, l'efficacité ayant un maximum, le rayonnement laser émis ayant une puissance de sortie optique ;

dans lequel le dispositif de commande de laser est configuré pour ajuster la puissance de sortie optique du rayonnement laser émis à une valeur cible sélectionnée en ajustant une température de fonctionnement de la source laser et/ou une température de fonctionnement du convertisseur de fréquence de telle sorte que le convertisseur de fréquence fonctionne à une efficacité plus faible que le maximum de l'efficacité.

2. Système laser médical selon la revendication 1, dans lequel ledit rayonnement a une longueur d'onde visible a un facteur de qualité de faisceau $M^2$ de moins de 3.

3. Système laser médical selon l'une quelconque des revendications précédentes ; comprenant en outre :

- un ou plusieurs actionneurs de concentration pour ajuster un point focal du rayonnement laser émis ou du rayonnement de sortie ;
- un ou plusieurs actionneurs de direction pour ajuster une partie d'émission de lumière du système laser de manière à diriger sélectivement le rayonnement émis ou le rayonnement de sortie vers différentes zones cibles à différentes distances de la partie d'émission de lumière ; et
- un dispositif de commande configuré pour commander le système laser médical pour

a) émettre un faisceau concentré vers une première zone cible ; dans lequel les un ou plusieurs actionneurs de concentration sont réglés de manière à concentrer le faisceau concentré à une première distance focale ;
b) ajuster les un ou plusieurs actionneurs de direction de manière à diriger le faisceau concentré vers une deuxième zone cible, et pour
c) émettre le faisceau concentré vers la deuxième zone cible sans reconcentrer le rayonnement émis ou le rayonnement de sortie.

4. Système laser médical selon l'une quelconque des revendications précédentes ; dans lequel le dispositif de commande de laser est configuré pour :

- déterminer une puissance de sotie optique émise par le système laser,
- comparer la puissance de sortie optique déterminée émise par le système laser à une valeur cible sélectionnée,
- ajuster la température de fonctionnement du convertisseur de fréquence sur la base de la comparaison de la puissance de sortie optique déterminée émise par le système laser à la valeur cible sélectionnée de manière à réduire l'amplitude de la différence entre la puissance de sortie optique déterminée et la valeur cible sélectionnée.

5. Système laser médical selon l'une quelconque des revendications précédentes ; dans lequel le dispositif de commande de laser est en outre configuré pour commander un paramètre de performance du rayonnement laser émis en commandant un courant d'injection vers la source laser.

6. Système laser médical selon l'une quelconque des revendications précédentes ; dans lequel le dispositif laser comprend un laser à diode conique.

7. Système laser médical selon l'une quelconque des revendications 1 à 6 ; dans lequel le dispositif laser comprend :

- un substrat ;
- un oscillateur maître monté sur le substrat et configuré pour émettre un rayonnement maître ;
- un amplificateur optique monté sur le substrat et configuré pour recevoir et amplifier le rayonnement maître et pour délivrer en sortie le rayonnement amplifié comme rayonnement source ;

- un isolant optique monté sur le substrat dans un chemin de rayonnement du rayonnement maître entre l'oscillateur maître et l'amplificateur optique ;
- un ou plusieurs éléments optiques montés sur le substrat et configurés pour diriger le rayonnement maître depuis l'oscillateur maître dans l'isolant optique et/ou pour diriger le rayonnement maître depuis l'isolant optique vers l'amplificateur optique ;

dans lequel les un ou plusieurs éléments optiques sont montés sur le substrat par une ou plusieurs montures déformables.

8. Système laser médical selon l'une quelconque des revendications précédentes ; comprenant un capteur pour détecter la puissance de sortie optique du rayonnement laser émis ; et
dans lequel le dispositif de commande de laser est configuré pour ajuster la température de fonctionnement du convertisseur de fréquence et/ou de la source laser jusqu'à ce que la puissance de sortie optique détectée corresponde à la valeur cible sélectionnée.

9. Système laser médical selon l'une quelconque des revendications précédentes ; comprenant un obturateur pouvant être commandé par l'utilisateur pour bloquer sélectivement le rayonnement laser émis ; et
dans lequel le dispositif de commande de laser est configuré pour empêcher l'ouverture de l'obturateur durant l'ajustement de la puissance de sortie optique.

10. Système laser médical selon l'une quelconque des revendications précédentes ; dans lequel la valeur cible sélectionnée est une valeur pouvant être sélectionnée par l'utilisateur sélectionnable entre une valeur cible sélectionnable la plus élevée qui est entre supérieure à 500 mW et inférieure à 2 W ; et
dans lequel la valeur cible sélectionnable la plus basse est entre supérieure à 50 mW et inférieure à 500 mW.

11. Système laser médical selon l'une quelconque des revendications précédentes ; dans lequel le dispositif de commande de laser est configuré pour ajuster la température de fonctionnement du convertisseur de fréquence et/ou de la source laser de telle sorte que le convertisseur de fréquence fonctionne dans un régime dans lequel un changement de l'efficacité du processus de conversion en fonction de la température de fonctionnement du convertisseur de fréquence est d'au moins 10 % par Kelvin.

12. Système laser médical selon l'une quelconque des revendications précédentes ; dans lequel le dispositif de commande de laser est configuré pour ajuster la température de fonctionnement du convertisseur de fréquence et/ou de la source laser de telle sorte que le convertisseur de fréquence fonctionne dans un régime dans lequel l'efficacité du processus de conversion est supérieure à 2 % et inférieure à 95 % d'une efficacité de conversion maximale en fonction de la température de fonctionnement du convertisseur de fréquence et/ou de la source laser.

13. Système laser médical selon l'une quelconque des revendications précédentes ; dans lequel le dispositif de commande de laser est configuré pour ajuster la température de fonctionnement du convertisseur de fréquence et/ou de la source laser de telle sorte que la longueur d'onde du rayonnement source est déplacée depuis une longueur d'onde à laquelle une efficacité de conversion maximale est obtenue de plus de 10 pm et moins de 100 pm, telle que de plus de 20 pm et de moins de 100 pm.

14. Système laser médical selon l'une quelconque des revendications précédentes ; dans lequel le dispositif de commande de laser est configuré pour ajuster la température de fonctionnement du convertisseur de fréquence et/ou de la source laser de telle sorte que la température de fonctionnement du convertisseur de fréquence dévie à partir d'une température à laquelle une efficacité de conversion maximale est obtenue de plus de 0,1 K et de moins de 1 K.

15. Procédé pour ajuster la puissance de sortie optique d'un système laser dans un système laser médical à une valeur cible sélectionnée, tel qu'un système pour chirurgie ophtalmologique, tel qu'un système laser de photo-coagulation ou un autre système laser de stimulation photo-thermique, le système laser comprenant :

- une source laser émettant un rayonnement source dans un premier intervalle de longueur d'onde, telle qu'une source laser infrarouge émettant de la lumière infrarouge ;
- un convertisseur de fréquence ayant une entrée et une sortie, le convertisseur de fréquence étant configuré pour recevoir le rayonnement source depuis la source laser au niveau de l'entrée et convertir le rayonnement source en rayonnement converti dans un second intervalle de longueur d'onde, par exemple lumière visible, et la sortie étant configurée pour délivrer en sortie le rayonnement converti, et une puissance de sortie optique du rayonnement converti dépendant d'une température de fonctionnement du convertisseur de fréquence,
- un élément de commande, par exemple un élément chauffant et/ou un élément de refroidissement, servant à ajuster et à stabiliser la température de fonctionnement du convertisseur de fréquence,
- une sortie configurée pour permettre au rayonnement converti de sortir du système laser,

dans lequel le procédé comprend la commande active de la température du convertisseur de fréquence de manière à ajuster ou à stabiliser la puissance de sortie optique émise par le système laser en effectuant les étapes suivantes :

a. détermination de la puissance de sortie optique émise par le système laser,
b. comparaison de la puissance de sotie optique déterminée émise par le système laser à la valeur cible sélectionnée,
c. ajustement de la température de fonctionnement du convertisseur de fréquence sur la base de la comparaison en (b) de manière à réduire l'amplitude de la différence entre la puissance de sortie optique déterminée et la valeur cible sélectionnée.

*FIG. 1*

*FIG. 2*

FIG. 3

*FIG. 4*

*FIG. 5*

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 20040078029 A
- US 4917486 A
- US 6370168 B
- WO 2013135271 A
- US 06559464 B

### Non-patent literature cited in the description

- **BECKER, B. C. ; MACLACHLAN, R. A. ; LOBES, L. A. ; RIVIERE, C. N.** Semiautomated intraocular laser surgery using handheld instruments. *Lasers Surg. Med.,* 2010, vol. 42, 264-273
- **YANG, S. ; LOBES, L. A. ; MARTEL, J. N. ; RIVIERE, C. N.** Handheld-automated microsurgical instrumentation for intraocular laser surgery. *Lasers Surg. Med.,* 2015, vol. 47, 658-668
- **KELEMEN, M T ; WEBER, J ; KAUFEL, G ; BIHL-MANN, G ; MORITZ, R et al.** Tapered diode lasers at 976nm with 8W nearly diffraction limited output power. *Electronics Letters,* 01 September 2005, vol. 41.18
- **JENSEN, O.B. ; HANSEN, A.K. ; MULLER, A. ; SUMPF, B. ; UNTERHUBER, A. ; DREXLER, W. ; PETERSEN, P.M. ; ANDERSEN, P.E.** Power Scaling of Nonlinear Frequency Converted Tapered Diode Lasers for Biophotonics. *Selected Topics in Quantum Electronics, IEEE Journal of,* March 2014, vol. 20 (2), 307-321
- **SUMPF, B. ; HASLER, K.-H. ; ADAMIEC, P. ; BUGGE, F. ; DITTMAR, F. ; FRICKE, J. ; WENZEL, H. ; ZORN, M. ; ERBERT, G. ; TRANKLE, G.** High-Brightness Quantum Well Tapered Lasers. *Selected Topics in Quantum Electronics, IEEE Journal,* May 2009, vol. 15 (3), 1009-1020